# EUROPEAN PATENT SPECIFICATION

(11) **EP 0 539 584 B1**
(45) Date of publication and mention of the grant of the patent: **20.11.1996**
(21) Application number: 90910922.5
(22) Date of filing: 18.07.1990
(51) Int. Cl.: C12P 21/08, G01N 33/569, A61K 39/395

(54) **REAGENT FOR DIAGNOSING MYCOPLASMA PNEUMONIAE**
REAGENS ZUR DIAGNOSE VON MYCOPLASMA PNEUMONIE
REACTIF PERMETTANT DE DIAGNOSTIQUER MYCOPLASMA PNEUMONIAE

(43) Date of publication of application: 05.05.1993
(73) Proprietor: DOJIN IYAKU-KAKO CO., LTD., Nakano-ku, Tokyo 164 (JP)
(72) Inventor: NAKAMURA, Yoshiko, 3130-10, Kobuchi, Kanagawa 229 (JP); SATO, Junichi, 2-20-13, Yatsu, Chiba 275 (JP)
(74) Representative: Wächtershäuser, Günter, Prof. Dr.
(86) International application number: JP9000926
(87) International publication number: WO9201808

(56) References cited:
- JP-A-57 145 818
- JP-A-61 076 423
- JP-A-62 289 523
- JP-A-63 184 064
- DATABASE WPI Section Ch, Week 8537, Derwent Publications Ltd., London, GB; Class A96, AN 85-226624 & JP-A-60 146 833 (GREEN CROSS CORP) 2 August 1985

## Description

### BACKGROUND OF THE INVENTION

### Field of the Invention:

The present invention relates to a diagnostic reagent for mycoplasma pneumoniae comprising an anti-mycoplasma pneumoniae monoclonal antibody or its labeled antibody in a stable manner.

### Background Art:

Mycoplasma pneumoniae infection diseases become prevalent almost every 4 years. Especially in infant and young patients, the diseases sometimes involve heavy symptoms and accompany serious complications such as disorders of the central nerve system and the like. Among cold syndrome, mycoplasma pneumoniae infection is popular next to virus infection, and macrolide or tetracycline antibiotics are known to be effective for its treatment. A prompt and accurate diagnosis of the disease at an early stage is therefore important. Methods of diagnosis of mycoplasma pneumoniae infection diseases adopted today are culture methods, serological methods, and the like. A problem with all of these methods is requiring a long time for the determination. Especially, the serological verification has drawbacks in that it can detect the antibody for the first time at the convalescence stage and it involves quite a few non-specific reactions. The present inventors have conducted studies in order to solve these drawbacks, and previously discovered an anti-mycoplasma pneumoniae monoclonal antibody having a specificity to mycoplasma pneumoniae and its usefulness as a diagnosis reagent of mycoplasma pneumoniae infection diseases (Japanese Patent Laid-open (ko-kai) No. 184064/1988).

However, the method of obtaining said anti-mycoplasma pneumoniae monoclonal antibody and the diagnosis reagent comprising the same were proven to have several problems. The purification of antibodies is generally carried out by the immunoglobulin purification methods, such as the ammonium sulfate fractionation method, ion-exchange chromatography, gel filtration, or the like; or the affinity chromatography using protein A or antigens. The present inventors also obtained said anti-mycoplasma pneumoniae monoclonal antibody by a purification method combining said ammonium sulfate fractionation and the protein A Sepharose® CL-4B (a product of Pharmacia) as described in the specification of said Japanese Patent Laid-open (ko-kai). The method was, however, accompanied by the following serious drawbacks in producing a large amount of the anti-mycoplasma pneumoniae monoclonal antibody.
1) The yield afforded by the ammonium sulfate fractionation is very low; no sufficient amount of antibody precipitate is obtained even at a 50% saturated ammonium sulfate concentration.
2) Since the antibody has a poor solubility in a neutral buffer solution, it is difficult to dissolve the ammonium sulfate precipitate in a small amount of buffer solution in a high concentration. It exhibits even a poorer solubility toward a low buffer solution with a low salt concentration, producing a large amount of precipitate by the dialysis and resulting in the deactivation.
3) Protein A Sepharose® 4B is expensive.

Because of these problems, it was difficult to purify the anti-mycoplasma pneumoniae monoclonal antibody which was discovered by the present inventors by a conventional purification method used for normal antibodies belonging to IgG.

In addition, the anti-mycoplasma pneumoniae monoclonal antibody obtained was unstable in normal storing conditions, posing a problem to develop this antibody as a diagnosis reagent.

JP-60/146 833 discloses a freeze-dried antibody preparation containing 1 part by weight of monoclonal antibody and as stabilizer 0.1-10 parts by weight of albumin. JP-62/289 523 discloses a composition containing an immunoglobulin and a stabilizer consisting of at least one saccharide and albumin.

A desire has therefore existed for the development of a purification method of the anti-mycoplasma pneumoniae monoclonal antibody suitable for mass production and a diagnosis reagent wherein said monoclonal antibody is kept stable.

In view of this situation, the inventors of the present invention have undertaken earnest studies. As a result, the inventors have found that the purification of the anti-mycoplasma pneumoniae monoclonal antibody can easily be achieved by concentrating a culture supernatant of a hybridoma which produces said monoclonal antibody, subjecting the concentrate to salting-out, and adjusting the pH in a certain range, and further that a stable diagnostic reagent can be obtained by dissolving the monoclonal antibody thus obtained together with albumin in a buffer solution having a specific pH or further by freeze-drying it. These findings have led to the completion of the present invention.

### Disclosure of the Invention

The present invention is to provide a method of stabilizing an anti-mycoplasma pneumoniae monoclonal antibody characterized by dissolving said anti-mycoplasma pneumoniae monoclonal antibody or its labeled antibody in an aqueous solution of pH 5.5-8.5 and containing albumin in an amount of 5-5,000 times by weight of said antibody or said labeled antibody or further by freeze-drying it; and a diagnostic reagent for mycoplasma pneumoniae (hereinafter abbreviated as M.p.) consisting essentially of an anti-mycoplasma pneumoniae monoclonal antibody or its labeled antibody and albumin in an amount of 5-5,000 times by weight of said antibody or said labeled antibody.

### Brief Description of the Drawings

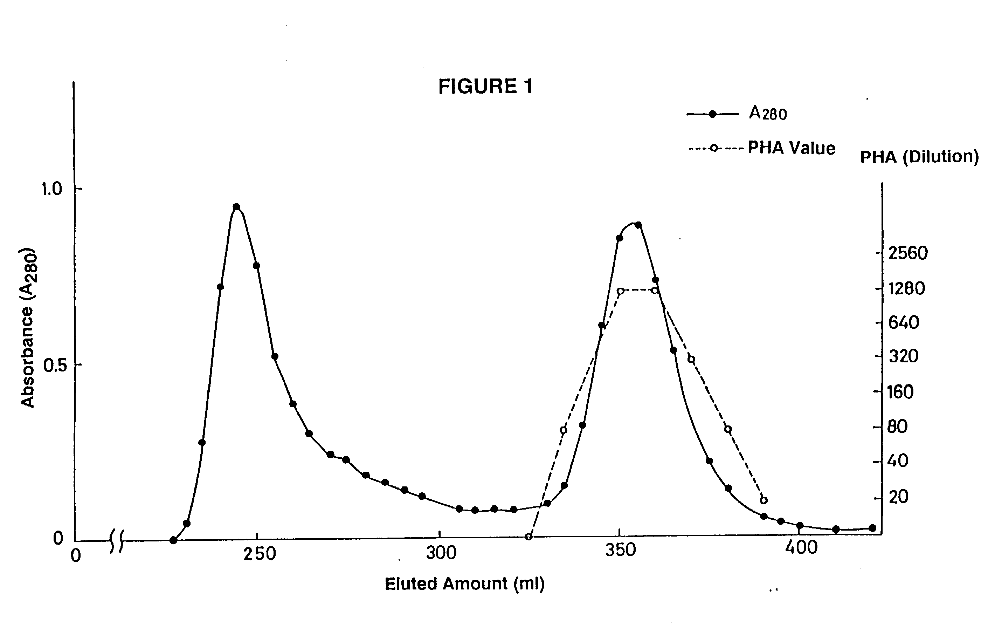

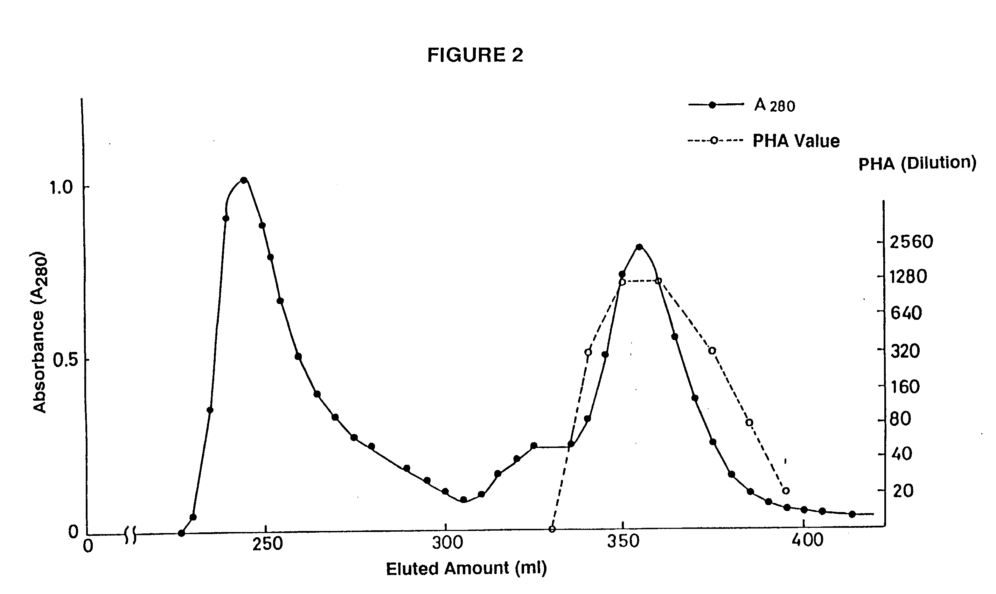

Figure 1 shows a gel filtration pattern of the ammonium sulfate fraction washed with a neutral buffer solution according to Example 3; and Figure 2 shows a gel filtration pattern of the unwashed ammonium sulfate fraction in Example 3.

### Best Mode for Implementing the Invention

The culture supernatant of a hybridoma used for the purification of the monoclonal antibody can be obtained, for example, by the method described in Japanese Patent Laid-open (ko-kai) No. 184064/1988. That is, a hybridoma (e.g., C2-G3, G1-B8, G1-E6) obtained by the cell fusion of immunocompetent cells and myeloma cells from mammal according to a conventional method is cultivated in a suitable medium, or the hybridoma is collected from ascites of an animal to which the hybridoma was intraperitoneally injected.

For carrying out the purification process of the present invention, the culture supernatant is first adjusted to pH 8-9 and concentrated to a concentration of 2-20 fold, preferably 5-10 fold. There are no specific limitations as to the method of concentration so long as such method does not produce precipitate, with a preferable method being ultrafiltration or dialysis. The concentrated culture supernatant is then salted out to collect the precipitate. The salting-out can be carried out, for example, by the ammonium sulfate fractionation method. That is to say, an equivalent amount of a saturated ammonium sulfate solution (50% saturation) adjusted to pH 7 is added to the concentrate, the mixture is allowed to stand still overnight, and the precipitate produced is dissolved in a 0.5 M carbonate buffer solution (pH 9.5), followed by the addition of a saturated ammonium sulfate solution (33% saturation) of a half amount, to collect the precipitate.

Next, the precipitate thus obtained is washed with a small amount of a buffer solution of pH 6.0-7.5, for example, with a 0.05 M phosphate buffer solution (pH 7.2), and dissolved into a buffer solution of pH 8-10, for example, into a 0.5 M carbonate buffer solution (pH 9.5), to obtain the high purity monoclonal antibody. The monoclonal antibody obtained according to this method has a considerably high concentration at this stage. It is preferred, however, to further purify it by gel filtration in order to obtain it in a higher purity. Here, other purification methods, for example, the purification method requiring the adsorption in a neutral range and at low salt concentration, e.g., the weak ion-exchange chromatography, is applied with difficulty. Also, from the aspect of stability, application of a purification method requiring an acidic state for the adsorption and elution is difficult. Gel filtration is preferably carried out by using a suitable carrier such as Sephacryl® S-300 HR, taking the molecular weight of IgG into consideration and by using a 0.5 M carbonate buffer solution (pH 9.5). The purified monoclonal antibody can be obtained by collecting the active fractions and concentrating them by the ultrafiltration. This purified antibody exhibits a uniform band in the electrophoresis. If dialyzed with a buffer solution having a stronger acidity than pH 9, the purified antibody produces a large amount of precipitate, rendering it difficult to handle. The above procedures are carried out preferably in a cold place.

The monoclonal antibody thus obtained exhibits a specificity toward M.p., thus it can be used as a diagnosis reagent for M.p. infection diseases as is, or labeled by a fluorescent pigment (e.g., fluorescence), an enzyme (e.g, peroxidase), a radiation substance (e.g., ¹²⁵I), a gold colloid, a latex, or the like. The measurement is carried out by the fluorescent analysis, the enzyme immunoassay, the radio immunoassay, the gold particle immunoassay, the coagulation reaction, or the like. Although the monoclonal antibody of the present invention is unstable and must be stored in a cold place, if the monoclonal antibody or its labeled antibody is dissolved in an aqueous solution with pH 5.5-8.5, preferably pH 6.0-8.0, and containing albumin in an amount of 5-1,000 times, preferably 10-500, by weight of said antibody or said labeled antibody; or if an aqueous solution with pH 5.5-8.5, preferably pH 6.0-8.0, and containing albumin in an amount of 5-5,000 times, preferably 50-5,000, by weight of said antibody or said labeled antibody is freeze-dried, the monoclonal antibody becomes highly stable and can be stored at room temperature. For example, the stability of the FITC-labeled monoclonal antibody can be promoted so that it can be stored at room temperature by adding 1.0-20 mg/ml, preferably 2.0-5.0 mg/ml, of albumin to a 10-200 µg/ml aqueous solution of the FITC-labeled monoclonal antibody with a pH of 5.5-8.5, preferably 6.0-8.0, or by adding 5.0-50 mg/ml, preferably 10-50 mg/ml, of albumin and freeze-drying it.

There are no specific limitations as to the albumin used in the present invention. Serum albumin, especially bovine serum albumin (hereinafter abbreviated as BSA), is preferable.

Since albumin does not interfere with the reaction of the monoclonal antibody of the present invention, the monoclonal antibody of the present invention or its labeled antibody, or a composition containing albumin in an amount of 5-5,000 times by weight of said monoclonal antibody or its labeled antibody thus obtained can be used as a stable M.p. diagnostic reagent.

### Examples

The present invention is hereinafter illustrated in more detail by way of examples.

### Reference Example

### <Preparation of Hybridoma>

### (1) Preparation of antibody producing cells

(i) Mycoplasma pneumoniae (Mac) cells were inoculated in the PPLO liquid medium and precultured at 37°C for 5 days. The culture liquid was inoculated into 1,000 ml of the same medium, and each 100 ml of the medium was transferred to 500 ml Roux flasks and allowed to stand still at 37°C for 5 days to culture the cells. After the cultivation, the culture liquid was discarded, and after the addition of a 0.01 M phosphate buffered saline (pH 7.2; hereinafter abbreviated as PBS), cells attached to the glass surface were scraped and collected by a rubber policeman. Freezing and thawing were repeated 4 times on this cell liquid, followed by washing 3 times with PBS to prepare M.p. membrane antigen (1). The protein concentration of this antigen was determined according to the method of Lowry et al. [Lowry, O. H., Rosenbrough, N. J., Farr, A. L., and Randall, R. J., J. Biol. Chem., 193, 265-275 (1951)]
(ii) In the same manner as above, Mac cells were inoculated in the EY medium (containing 2% egg yolk) reported by Sasaki et al and precultured at 37°C for 5 days. The culture liquid was inoculated into 1,000 ml of the same medium and allowed to stand still at 37°C for 5 days to culture the cells. After the cultivation, the culture liquid was discarded, and after the addition of PBS, cells attached to the glass surface were scraped and collected by a rubber policeman. Freezing and thawing were repeated 4 times on this cell liquid, followed by washing 3 times with PBS to prepare M.p. membrane antigen (2). The protein concentration of this antigen was determined according to the method of Lowry et al.
(iii) M.p. membrane antigen (1) (100 µg as protein) was intraperitoneally injected as is to BALB/c female mouse (age: 6-7 weeks). After 1 week, additional immunization was performed by the intraperitoneal injection of the same amount. Furthermore, 6 weeks thereafter, M.p. membrane antigen (2) of the same amount was intravenously injected. After 1-2 weeks, upon confirmation of the increase in the antibody titer in the blood, M.p. membrane antigen (2) (10 µg as protein) was intravenously injected.
(iv) The spleen was aseptically taken out 3 days after the final immunization and loosened by dressing forceps to prepare a cell suspension by passing the cells through # mesh. After the addition of 10 fold of a Tris ammonium chloride buffer solution, the cell suspension was allowed to stand still for 2-3 minutes in ice-water to remove erythrocytic cells. After washing 3 times with Eagle's MEM medium (hereinafter abbreviated as MEM), cells were adjusted to 5.0x10⁷ cells/ml.

### (2) Cell Fusion and Preparation of Antibody-producing Hybridoma

(i) Myeloma cells (X63-Ag 8.6.5.3) which had been cultivated in advance were washed 3 times with MEM and adjusted to 5.0x10⁶ cells/ml. 5.0 ml of spleen cells prepared in (1) (iv) above and 5.0 ml of the myeloma cells were placed in a 40 ml centrifuge tube and centrifuged at 1,100 rpm for 5 minutes to remove the supernatant and to collect cells. The pellet was well loosened and 0.5 ml of a 50% solution of polyethylene glycol 1000 warmed to 37°C in advance was gradually charged into the cells over a period of about 1 to 2 minutes. Then, 10 ml of MEM which had been warmed to 37°C in advance was gradually charged at a rate of about 2 ml/min to terminate the reaction. After removing the supernatant by centrifugation (1,100 rpm, 5 minutes), RPMI-1640 medium containing 10% BSA was added to adjust the concentration of myeloma cells to 5.0x10⁵/ml. The mixture was dispensed into a 96 well microplate in an amount of 200 µl/well. After cultivation at 37°C in a carbon dioxide cultivator containing 5% of carbon dioxide for 1 day, one half of the medium was discharged and 100 µl of HAT medium (RPMI-1640 medium containing 10% BSA added with 1.0x10⁻⁴ M hypoxanthine, 4.0x10⁻⁷ M aminopterin, and 1.6x10⁻⁵ M thymidine). Thereafter, one half amount of the medium was replaced by the HAT medium once every 2 or 3 days. After 10-14 days, when proliferation of cells was observed, one half of the medium was replaced by HT medium (a medium excluding aminopterin from HAT medium). After 2 days of the replacement by HT medium, the medium was replaced by RPMI-1640 medium containing 10% BSA once every 2 to 3 days. After about 2 weeks, anti-M.p. antibody-producing hybridoma was screened by the ELISA method and the indirect erythrocytic cell coagulation reaction (Cellodiar MYCO, a product of Fuji Revio Co. was used). As a result fusion cells were found in all wells and production of the antibody was confirmed in 6.8% of these wells. Only 3.7% of the wells exhibited reproducibility after the cultivation was completed.
(ii) The hybridomas on which the antibody production was recognized were cloned by the limiting dilution method. That is, the hybridomas adjusted to 6.0 cells/ml were mixed with an equivalent amount of a liquid containing 10⁷/ml thymocites from a normal BALB/c mouse and poured into a 96 well microplate in an amount of 200 µl per well. After cultivation at 37°C in a carbon dioxide cultivator containing 5% of carbon dioxide for about 2-3 weeks, the production of antibody in the culture supernatant was investigated according to the same manner as above. Cloning was repeated on hybridomas exhibiting good production of the antibody. In order to collect the antibody from the culture supernatant and to confirm stable production of the antibody for a long period of time, hybridomas obtained by cloning two times were transferred from the 96 well microplate to a 24 well multiplate and then to a 50 ml tissue culture flask. As a result, stable antibody production was confirmed to be possible over generations for a long period of time.

Hybridomas C2-G3, G1-B8, and G1-E6 were thus obtained.

### Example 1 <Purification of Monoclonal Antibody>

Hybridoma G1-E6 obtained in Reference Example was cultivated in GIT medium (a product of Nissui Pharmaceutical Co.) at 37°C in a carbon dioxide cultivator containing 5% of carbon dioxide. After about 4-5 days, the culture supernatant was collected (1,500 ml), adjusted to pH 8, centrifuged (10,000 rpm, 20 minutes), and filtered through a membrane filter. The filtrate was concentrated by a ultrafilter to a 5 fold concentration. An equivalent amount of saturated ammonium sulfate (SAS; 50% saturation) adjusted to pH 7 was added to the concentrate, left in a cold place overnight, and centrifuged (10,000 rpm, 20 minutes) to collect the precipitate. The precipitate was dissolved in 200 ml of a 0.5 M carbonate buffer solution (pH 9.5), and 100 ml of SAS (33% saturation) was added to the solution. The solution was allowed to stand in a cold place to collect the precipitate produced by centrifugation (10,000 rpm, 20 minutes). The precipitate was washed with 10 ml of a 0.05 M phosphate buffer solution (pH 7.2) and centrifuged (10,000 rpm, 20 minutes) to collect the precipitate. This precipitate was suspended in 8 ml of a 0.5 M carbonate buffer solution (pH 9.5) and dialyzed against a 0.5 M carbonate buffer solution (pH 9.5) overnight. After removing a slight amount insoluble components which remained by centrifuge (10,000 rpm, 20 minutes), the solution was fractionated by the gel filtration method using a column (diameter: 26 mm, length: 1,000 mm) packed with Sephacryl® S-300 HR (manufactured by Pharmacia) using a 0.5 M carbonate buffer solution (pH 9.5). The active fractions were collected and concentrated by ultrafiltration to obtain purified antibody (yield: 35.6 mg). The purified antibody was electrophoretically homogeneous.

### Comparative Example 1 <Purification of Monoclonal Antibody (method of Japanese Patent Laid-open (ko-kai) No. 184064/1988)>

To 1,500 ml of the same hybridoma culture supernatant as used in Example 1 an equivalent amount of SAS adjusted to pH 7 was added and the mixture was allowed to stand still in a cold place overnight, followed by centrifugation (10,000 rpm, 20 minutes) to collect the precipitate. The precipitate was dissolved into 250 ml of 0.05 M Tris buffered saline (pH 8.6) and dialyzed overnight. After removing insoluble components by centrifugation (10,000 rpm, 20 minutes), the solution was fractionated by the affinity chromatography using a column (diameter: 26 mm, length: 400 mm) packed with Protein A-Sepharose® 4B (manufactured by Pharmacia) using 0.05 M Tris buffered saline (pH 8.6). The active fractions were collected and concentrated by ultrafiltration to obtain the purified antibody (yield: 1.3 mg).

### Example 2

The culture supernatant of Example 1 was concentrated by ultrafiltration in various concentrations. To the concentrates 1/2 or an equivalent amount of SAS (33% saturation or 50% saturation) was added. The recovery rates of the antibody contained in the precipitate thus produced were compared. The results are shown in Table 1.

**TABLE 1**

| Comparison of Antibody Recovery Rates from Hybridoma Culture Supernatant by the Ammonium Sulfate Precipitation | | |
|---|---|---|
| Concentration Rate of Culture Supernatant | | Rate of Recovery (%) |
| Not concentrated | (33% saturation) | 10 |
| Not concentrated | (50% saturation) | 17 |
| 2-fold concentration | (50% saturation) | 25 |
| 5-fold concentration | (33% saturation) | 33 |
| 5-fold concentration | (50% saturation) | 87 |
| 10-fold concentration | (33% saturation) | 50 |
| 10-fold concentration | (50% saturation) | 83 |

### Example 3

In the course of purification of the monoclonal antibody described in Example 1, the 33% SAS precipitate obtained from the culture supernatant was washed with a neutral buffer solution to investigate the effect of the purification.

A solution of the 50% SAS precipitate in a carbonate buffer solution of pH 9.5 was divided into 2 portions. To each portion was added 1/2 amount of SAS to obtain 33% saturation SAS precipitate. One of the portions was dissolved as is into a carbonate buffer solution of pH 9.5 (1), and the other was washed with a phosphate buffer solution of pH 7.2 (2), then dissolved into a carbonate buffer solution of pH 9.5 (3). The antibody specific activity and the rate of recovery of each solution were compared. The results are shown in Table 2.

**TABLE 2**

| Effects of Purification by Washing 33% Saturation SAS Precipitate in a Neutral Buffer Solution | | |
|---|---|---|
| Purification Stage | Specific Activity * | Rate of Recovery (%) |
| (1) 33% Saturation SAS Precipitate | 630 | 100 |
| (2) Washed by pH 7.2 Phosphate Buffer Solution | 350 | 12 |
| (3) Washed by pH 9.5 Carbonate Buffer Solution | 820 | 88 |

| | | |
|---|---|---|
| * Activity unit/Protein Amount (mg) | | |

The ammonium sulfate fractions washed with a neutral buffer solution (3) and the ammonium sulfate fractions without washing (1) were purified, respectively, by the gel filtration method. The separation patterns for each purification are shown in Figures 1 and 2, wherein the PHA value is a coagulation activity when Cellodier MYCO-II (manufactured by Fuji Revio Co.) was used. The antibody separation was more clear when the ammonium sulfate fractions washed with a neutral buffer solution were used.

### Example 4 <Preparation of Fluorescent-Labeled Monoclonal Antibody>

Four fold amount, in molar ratio, of FITC (Fluorescein isothiocyanate) was added to 5 mg of the purified antibody obtained in Example 1. The mixture was reacted in a 0.5 M carbonate buffer solution (pH 9.5) at 4°C for 4 hours. After the reaction, insoluble components were centrifugally separated (10,000 rpm, 10 minutes), followed by removal of unreacted FITC by gel filtration using a column (diameter: 10 mm, length: 400 mm) packed with Sephadex® G-25 (manufactured by Pharmacia) using 150 mM sodium chloride to obtain purified fluorescent-labeled monoclonal antibody (yield: 4.5 mg). The ratio of FITC bound to 1 molecule of the antibody (F/P ratio) was 1.1. Almost all FITC was confirmed to have bound to the H-chain in the antibody molecule by means of the SDS-electrophoresis.

### Example 5 <Preparation of Peroxidase-Labeled Monoclonal Antibody>

4 mg of sodium periodide was added to a horseradish peroxidase solution (4 mg/ml) to react at room temperature for 10 minutes. After the reaction, the solution was dialyzed against a 1 mM acetate buffer solution (pH 4.0) at 4°C overnight. An aldehyde peroxidase solution thus obtained was adjusted to pH 9.3 by a 0.2 M carbonate buffer solution (pH 9.5), immediately followed by the addition of 5 mg of the purified antibody (dialyzed against a 0.01 M carbonate buffer solution of pH 9.5 at 4°C overnight) obtained in Example 1. After the reaction at room temperature for 2 hours, 0.4 mg sodium hydrogen borate was added, and the mixture was reacted for 2 hours at 4°C and centrifuged (10,000 rpm, 10 minutes) to remove insoluble components. High molecular weight polymers of peroxidase and antibody, unreacted peroxidase, and unreacted antibody were removed by gel filtration using a column (diameter: 16 mm, length: 700 mm) packed with Ultrogel ACA-44 (manufactured by LKB Co.) using a 0.01 M carbonate buffer (pH 9.5)-150 mM sodium chloride solution to obtain purified peroxidase-labeled monoclonal antibody (yield: 4.5 mg).

### Example 6 <Preparation of Biotin-Labeled Monoclonal Antibody>

1 mg of N-hydroxysuccinimide biotin (a product of Funakoshi Pharmaceutical Co.) was dissolved into 50-100 µl of dimethylformamide, followed by the addition of 5 mg of the purified antibody (adjusted to 1 mg/ml and dialyzed against 0.1 M sodium hydrogen carbonate at 4°C overnight) obtained in Example 1. The mixture was reacted at room temperature for 4 hours. After the reaction, insoluble components were centrifugally separated (10,000 rpm, 10 minutes). Unreacted N-hydroxysuccinimide biotin was removed by gel filtration using a column (diameter: 16 mm, length: 400 mm) packed with Ultrogel ACA-44 (manufactured by LKB Co.) using a 0.01 M carbonate buffer-0.15 M sodium chloride solution to obtain purified biotin-labeled monoclonal antibody (yield: 4.1 mg).

### Example 7 <Effects of pH on Thermal Stability of Fluorescent-Labeled Antibody>

A solution containing 80 µg of the FITC-labeled monoclonal antibody obtained in Example 4, 20 mg of BSA, and 8.5 mg of sodium chloride, each in 1 ml, was adjusted to various pHs. These solutions were heated at 50°C for 12 hours or at 40°C for 1 week to compare their stabilities. The results are shown in Table 3.

**TABLE 3**

| Effects of pH on Thermal Stability of Fluorescent-Labeled Antibody | | |
|---|---|---|
| (Residual activity %) | | |
| pH | Heated at 50°C for 12 hours | Heated at 40°C for 1 week |
| 5.0 | 13% | 46% |
| 5.5 | 38% | 78% |
| 6.0 | 64% | 82% |
| 6.5 | 56% | 83% |
| 7.0 | 47% | 71% |
| 7.5 | 47% | 74% |
| 8.0 | 46% | 64% |
| 8.5 | 28% | 63% |
| 9.0 | 13% | 50% |
| 9.5 | 0% | 29% |

### Example 8 <Effects of BSA on Thermal Stability of Fluorescent-Labeled Antibody Solution>

Solutions (pH 6.5) containing 80 µg of the FITC-labeled monoclonal antibody obtained in Example 4, 1.0-50 mg of BSA and 8.5 mg of sodium chloride, each in 1 ml, were prepared. These solutions were heated at 50°C for 12 hours or at 40°C for 1 week to compare their stabilities with the stability of a solution not containing BSA. The results are shown in Table 4. The monoclonal antibody was confirmed to be stable in solutions containing 1.0-20 mg/ml BSA, and particularly stable in a solution containing 2.0-5.0 mg/ml of BSA; i.e., stable for an amount of BSA of 12.5-250 times by weight, particularly 25-62.5 times by weight, of the monoclonal antibody.

**TABLE 4**

| Effects of BSA on Thermal Stability of Fluorescent-Labeled Antibody | | |
|---|---|---|
| BSA Content (mg/ml) | Heated at 50°C for 12 hours | Heated at 40°C for 1 week |
| 0 | 50% | 74% |
| 1 | 67% | 99% |
| 2 | 77% | 100% |
| 5 | 86% | 99% |
| 10 | 73% | 97% |
| 20 | 66% | 86% |
| 50 | 53% | 73% |

### Example 9 <Effects of BSA on Thermal Stability of Fluorescent-Labeled Freeze-dried Antibody>

Solutions (pH 6.5) containing 80 µg of the FITC-labeled monoclonal antibody obtained in Example 4, 5.0-50 mg of BSA, and 8.5 mg of sodium chloride, each in 1 ml, were prepared. Each solution (0.5 ml) was poured into a brown vial and freeze-dried. Vials were replaced by nitrogen gas (each vial contained 40 µg of the FITC-labeled monoclonal antibody, 2.5-25 mg of BSA, and 4.25 mg of sodium chloride). The vials were heated at 50°C for 1 week to compare their stabilities with the stability of a vial not containing BSA. The results are shown in Table 5.

The monoclonal antibody was confirmed to be stable in vials containing 2.5-25 mg of BSA, and particularly stable in vials containing 5.0-25 mg of BSA; i.e., stable for an amount of BSA of 62.5-625 times by weight, particularly 125-625 times by weight, of the monoclonal antibody.

**TABLE 5**

| Effects of BSA on Thermal Stability of Fluorescent-Labeled, Freeze-dried Antibody | |
|---|---|
| Residual activity (%) | |
| BSA Content (mg/vial) | Heated at 50°C for 1 week |
| 0 | 3% |
| 2.5 | 35% |
| 5.0 | 61% |
| 10 | 88% |
| 25 | 78% |

### Example 10 <Diagnostic Reagent for Mycoplasma Pneumoniae comprising Fluorescent-Labeled Monoclonal Antibody>

(i) 8 mg of the FITC-labeled monoclonal antibody obtained in Example 4, 500 mg of BSA, 850 mg of sodium chloride, and 50 mg of sodium azide were dissolved in 100 ml of a 50 mM phosphate buffer solution (pH 7.0). The solution was dispensed into brown vials, each in an amount of 1 ml.
(ii) 2 mg of the FITC-labeled monoclonal antibody obtained in Example 4, 500 mg of BSA, 850 mg of sodium chloride, and 50 mg of sodium azide were dissolved in 100 ml of a 50 mM phosphate buffer solution (pH 8.0). The solution was dispensed into brown vials, each in an amount of 1 ml.
(iii) 8 mg of the FITC-labeled monoclonal antibody obtained in Example 4, 2,000 mg of BSA, 850 mg of sodium chloride, and 50 mg of sodium azide were dissolved in 100 ml of a 50 mM phosphate buffer solution (pH 8.0). The solution was dispensed into brown vials, each in an amount of 0.5 ml, and freeze-dried. The vials were replaced by nitrogen gas.

The stabilities of diagnostic reagents for mycoplasma pneumoniae comprising fluorescent-labeled monoclonal antibody prepared in (i), (ii), and (iii) above are shown in Table 6.
Comparative Example (i): A solution of the antibody (80 µg/ml) in a buffer solution of pH 9.5 to which no BSA was added.
Comparative Example (ii): A solution of the antibody (80 µg/ml) and BSA (5 mg/ml) in a buffer solution of pH 9.5 was dispensed in brown vials, 0.5 ml in each vial, the solution was freeze-dried, and vials were replaced by nitrogen gas.

**TABLE 6**

| Stability of Diagnostic Reagent for Mycoplasma Pneumoniae | | | | | |
|---|---|---|---|---|---|
| | Example | | | Comparative Example | |
| Storing Conditions | (i) | (ii) | (iii) | (i) | (ii) |
| One month in a refrigerator | 100 | 100 | 100 | 90 | 95 |
| One month at 25°C | 100 | 98 | 98 | 15 | 40 |
| One month at 40°C | 90 | 95 | 98 | 0 | 0 |

### Industrial Applicability of the Invention

A anti-mycoplasma pneumoniae monoclonal antibody exhibiting excellent specificity to mycoplasma pneumoniae can be obtained inexpensively, by a simple manner, at a high purity, in a high yield, and in a large amount. According to the present invention, stability of the anti-mycoplasma pneumoniae monoclonal antibody or its labeled antibody were able to be remarkably promoted, ensuring extreme utility as a diagnostic reagent.

## Claims

1. A diagnostic reagent for mycoplasma pneumoniae consisting essentially of an anti-mycoplasma pneumoniae monoclonal antibody or its labeled antibody and albumin, which is prepared by dissolving an anti-mycoplasma pneumoniae monoclonal antibody or its labeled antibody and albumin in an amount of 10-500 times by weight of the antibody or the labeled antibody in an aqueous solution of pH 5.5-8.5, or which is prepared by dissolving an anti-mycoplasma pneumoniae monoclonal antibody or its labeled antibody and albumin in an amount of 50-5000 times by weight of the antibody or the labeled antibody in an aqueous solution of pH 5.5-8.5 and freeze-drying the solution.

2. The diagnostic reagent for mycoplasma pneumoniae according'to Claim 1, which is prepared by dissolving 10-200 µg/ml of an anti-mycoplasma pneumoniae monoclonal antibody or its labeled antibody and 5.0-50 mg/ml of albumin in an aqueous solution of pH 5.5-8.5 and freeze-drying the solution.

3. The diagnostic reagent for mycoplasma pneumoniae according to Claim 1, which is prepared by dissolving 1-20 parts by weight of an anti-mycoplasma pneumoniae monoclonal antibody or its labeled antibody and 100-2,000 parts by weight of albumin in an aqueous solution of pH 5.5-8.5.

4. The diagnostic reagent for mycoplasma pneumoniae according to Claim 1, which is prepared by dissolving 10-200 µg/ml of an anti-mycoplasma pneumoniae monoclonal antibody or its labeled antibody in an aqueous solution of pH 5.5-8.5 containing 1.0-20 mg/ml of albumin.

5. A method of stabilizing an anti-mycoplasma pneumoniae monoclonal antibody, consisting essentially of dissolving an anti-mycoplasma pneumoniae monoclonal antibody or its labeled antibody in an aqueous solution of pH 5.5-8.5, consisting essentially of a buffer solution and albumin in an amount of 10-500 times by weight.

6. A method of stabilizing an anti-mycoplasma pneumoniae monoclonal antibody, comprising:
dissolving said anti-mycoplasma pneumoniae monoclonal antibody in an aqueous solution consisting essentially of a buffer solution of pH 5.5-8.5 and albumin in an amount of from 50 to 5000 times by weight of said antibody, and freeze-drying said solution.

## Patentansprüche

1. Diagnostisches Reagens für Mycoplasma pneumoniae, das im wesentlichen aus einem monoklonalen anti-Mycoplasma pneumoniae-Antikörper oder seinem markierten Antikörper und Albumin besteht, das durch Lösen eines monoklonalen anti-Mycoplasma pneumoniae-Antikörpers oder seines markierten Antikörpers und von Albumin in einer Menge des 10-500fachen des Gewichts des Antikörpers oder des markierten Antikörpers in einer wäßrigen Lösung von pH 5,5-8,5 hergestellt ist oder das durch Lösen eines monoklonalen anti-Mycoplasma pneumoniae-Antikörpers oder seines markierten Antikörpers und von Albumin in einer Menge des 50-5000fachen des Gewichts des Antikörpers oder des markierten Antikörpers in einer wäßrigen Lösung von pH 5,5-8,5 und Gefriertrocknen der Lösung hergestellt ist.

2. Diagnostisches Reagens für Mycoplasma pneumoniae nach Anspruch 1, das durch Lösen von 10-200 µg/ml eines monoklonalen anti-Mycoplasma pneumoniae-Antikörpers oder seines markierten Antikörpers und 5,0-50 mg/ml Albumin in einer wäßrigen Lösung von pH 5,5-8,5 und Gefriertrocknen der Lösung hergestellt ist.

3. Diagnostisches Reagens für Mycoplasma pneumoniae nach Anspruch 1, das durch Lösen von 1-20 Gewichtsteilen eines monoklonalen anti-Mycoplasma pneumoniae-Antikörpers oder seines markierten Antikörpers und 100-2000 Gewichtsteilen Albumin in einer wäßrigen Lösung von pH 5,5-8,5 hergestellt ist.

4. Diagnostisches Reagens für Mycoplasma pneumoniae nach Anspruch 1, das durch Lösen von 10-200 µg/ml eines monoklonalen anti-Mycoplasma pneumoniae-Antikörpers oder seines markierten Antikörpers in einer wäßrigen Lösung von pH 5,5-8,5, die 1,0-20 mg/ml Albumin enthält, hergestellt ist.

5. Verfahren zur Stabilisierung eines- monoklonalen anti-Mycoplasma pneumoniae-Antikörpers, das im wesentlichen aus dem Lösen eines monoklonalen anti-Mycoplasma pneumoniae-Antikörpers oder seines markierten Antikörpers in einer wäßrigen Lösung von pH 5,5-8,5, die im wesentlichen aus einer Pufferlösung und Albumin in einer Menge des 10-500fachen des Gewichts besteht, besteht.

6. Verfahren zur Stabilisierung eines monoklonalen anti-Mycoplasma pneumoniae-Antikörpers, umfassend:
Lösen des monoklonalen anti-Mycoplasma pneumoniae-Antikörpers in einer wäßrigen Lösung, die im wesentlichen aus einer Pufferlösung von pH 5,5-8,5 und Albumin in einer Menge des 50 bis 5000fachen des Gewichts des Antikörpers besteht, und Gefriertrocknen der Lösung.

## Revendications

1. Réactif de diagnostic pour mycoplasma pneumoniae, consistant essentiellement en un anticorps monoclonal anti-mycoplasma pneumoniae, ou son anticorps marqué, et en albumine, qui est préparé par dissolution d'un anticorps monoclonal anti-mycoplasma pneumoniae, ou de son anticorps marqué, et de l'albumine dans une quantité de 10-500 fois le poids de l'anticorps ou de l'anticorps marqué, dans une solution aqueuse de pH 5,5-8,5, ou qui est préparé par dissolution d'un anticorps monoclonal anti-mycoplasma pneumoniae, ou de son anticorps marqué, et de l'albumine dans une quantité de 50-5000 fois le poids de l'anticorps ou de l'anticorps marqué, dans une solution aqueuse de pH 5,5-8,5, et lyophilisation de la solution.

2. Réactif de diagnostic pour mycoplasma pneumoniae, selon la revendication 1, qui est préparé par dissolution de 10-200 µg/ml d'un anticorps monoclonal anti-mycoplasma pneumoniae, ou de son anticorps marqué, et 5,0-50 mg/ml d'albumine, dans une solution aqueuse de pH 5,5-8,5, et lyophilisation de la solution.

3. Réactif de diagnostic pour mycoplasma pneumoniae, selon la revendication 1, qui est préparé par dissolution de 1-20 parties en poids d'un anticorps monoclonal anti-mycoplasma pneumoniae, ou de son anticorps marqué, et 100-2000 parties en poids d'albumine, dans une solution aqueuse de pH 5,5-8,5.

4. Réactif de diagnostic pour mycoplasma pneumoniae, selon la revendication 1, qui est préparé par dissolution de 10-200 µg/ml d'un anticorps monoclonal anti-mycoplasma pneumoniae, ou de son anticorps marqué, dans une solution aqueuse de pH 5,5-8,5, contenant 1,0-20 mg/ml d'albumine.

5. Procédé de stabilisation d'un anticorps monoclonal anti-mycoplasma pneumoniae, consistant essentiellement à dissoudre un anticorps monoclonal anti-mycoplasma pneumoniae, ou son anticorps marqué, dans une solution aqueuse de pH 5,5-8,5, consistant essentiellement en une solution tampon et en albumine dans une quantité de 10-500 fois en poids.

6. Procédé de stabilisation d'un anticorps monoclonal anti-mycoplasma pneumoniae comprenant :
la dissolution dudit anticorps monoclonal anti-mycoplasma pneumoniae dans une solution aqueuse consistant essentiellement en une solution tampon de pH 5,5-8,5 et en albumine dans une quantité de 50 à 5000 fois le poids dudit anticorps, et la lyophilisation de ladite solution.
